# EUROPEAN PATENT SPECIFICATION

(11) **EP 4 377 283 B1**
(45) Date of publication and mention of the grant of the patent: **27.08.2025**
(21) Application number: 22755142.1
(22) Date of filing: 22.07.2022
(51) Int. Cl.: C07C 29/128, C07C 31/02

(54) **METHOD FOR THE EXTRACTION OF POLICOSANOLS FROM INDUSTRIAL HEMP AND MIXTURE THEREOF**
VERFAHREN ZUR EXTRAKTION VON POLIKOSANOLEN AUS INDUSTRIEHANF UND MISCHUNG DAVON
PROCÉDÉ D'EXTRACTION DE POLICOSANOLS DE CHANVRE INDUSTRIEL ET MÉLANGE DE CES DERNIERS

(30) Priority: 29.07.2021 IT 202100020228
(43) Date of publication of application: 05.06.2024
(73) Proprietor: Chromaleont S.r.l., 98165 Messina (IT); Sapienza Universita di Roma, 00185 Rome (IT)
(72) Inventor: LAURA CAPRIOTTI, Anna, 00146 Roma (IT); CAVALIERE, Chiara Cavaliere, 0144 Roma (IT); CERRATO, Andrea, 00182 Roma (IT); LAGANÀ, Aldo, 00146 Roma (IT); MICALIZZI, Giuseppe, 98043 Rometta (ME) (IT); MONDELLO, Luigi, 98165 Messina (IT); MONTONE, Carmela Maria, 00146 Roma (IT); PIOVESANA, Susy, 00158 Roma (IT)
(74) Representative: Longoni, Alessandra
(86) International application number: PCT/EP2022/070579
(87) International publication number: WO 2023/006593

(56) References cited:
- US-A- 5 856 316
- US-B1- 6 225 354
- US-B2- 7 214 394

## Description

The present invention relates to a method for the extraction of policosanols from industrial hemp (Cannabis sativa L.) and in particular from the waste issued from the cannabidiol extraction process. The present also relates to the mixture of policosanols obtained by this method.

Policosanols are long-chain primary alcohols, having 20 to 36 carbon atoms. They are present in free or esterified fatty acid form in numerous matrices, both of animal and vegetable origin, such as beeswax, rice bran, wheat germ, fruit and dried fruit.

The growing interest towards policosanols derives from the numerous benefits that their consumption brings to human health, as evidenced by many studies in the literature.

Specifically, policosanols show an anticholesterolemic activity, demonstrated by experiments both on animal models and on patients suffering from type 2 hypercholesterolemia, exerted by inhibition of the 3-hydroxy-3-methylglutatyl coenzyme A reductase enzyme, higher than that of other phytosterols and phytostanols (J.T. Chen et al., Pharmacotherapy 2005, 25 (2): 171-183; I. Gouni-Berthold et al., Am. Heart J. 2002, 143: 356-365.).

Policosanols contribute to the prevention and treatment of cardiovascular diseases (K.A. Varady et al., Nutr. Rev. 2003, 61 (2): 376-383.), to the reduction of the formation of atherosclerotic plaques (K.A. Varady et al., Nutr. Rev. 2003, 61 (2): 376-383.), have a protective activity in patients at risk of heart attacks (M. Noa et al., J. Med. Food. 2007, 10: 452- 459.), exhibit an anticoagulant effect and prevent lipofundin-induced atherosclerotic lesions (M. Noa et al., J. Med. Food. 2007, 10: 452-459.). Furthermore, these molecules have antioxidant activity and enhance tissue regeneration (H. Ham et al., J. Food Biochem. 2015, 40: 526-534; J. Shen et al., J. Funct. Foods. 2019,57: 351-360.)

Given their numerous properties, policosanols are increasingly being included in the formulations of nutraceuticals, pharmaceuticals, food and cosmetics.

The policosanols present in free form in natural matrices, such as seeds or other plant or animal material, are extracted by extraction with apolar solvents, often assisted by ultrasounds to speed up the extraction process and reduce the process temperature (J. Shen et al., J. Funct. Foods. 2019, 57: 351-360).

Alternatively, J. Shen et al. (J. Funct. Foods. 2019, 57: 351-360) describes a procedure that exploits supercritical CO₂ for the extraction of policosanols from different matrices, such as cloves, wheat straw, quince, perennial weeds of the genus Miscanthus , beeswax and sugar cane waxes, with high yields; this technique has also been used in combination with immobilized lipase catalysis to extract tetracosanol (C24), hexacosanol (C26), octacosanol (C28), triacontanol (C30) and dotriacontanol (C32) from beeswax (M.A. Jackson et al. , J. Supercrit. Fluids. 2006, 37: 173-177).

Policosanols can also be produced by hydrolysis starting from waxy material, where they are present in esterified form with fatty acids.

There are several known processes of extraction of policosanols from waxy material of both animal and vegetable origin.

For example, US patent 5856316 describes the extraction of policosanols from sugar cane wax by saponification for pharmaceutical applications. The described method provides a 94% pure policosanol blend with a yield of about 28%.

US patent 6225354 describes the extraction of policosanols from beeswax by solvent extraction for pharmaceutical applications. The method provides a 93-99% pure blend with a 35% yield.

Patent applications US 2006822004 and WO 2004/069161 describe the extraction of policosanols from Chinese wax, produced by insects (*Ericerus pela),* by hydrolysis and extraction to obtain low purity mixtures.

Patent IT 1331882 describes the production of policosanols from rice processing waste, by extracting the waste with solvent, hydrolysis of the extract and final extraction with solvent.

US patent 7214394 describes the isolation of policosanols from plant products (seed oils, soybean) by various procedures, including solvent extraction and transesterification, for the production of nutraceutical compounds.

Patent application WO 2015/128829 describes the production of policosanols from animal and vegetable waxes, by microwave-assisted transesterification and hydrolysis, obtaining pure mixtures at 90-100%.

In this context, among the numerous matrices studied, there are no studies describing the extraction of policosanols from industrial hemp (*Cannabis sativa L*.).

Hemp, in particular its inflorescences, is however widely used as a source of pharmacologically active compounds, for example cannabinoids, such as cannabidiol.

The industrial extraction process of cannabidiol from industrial hemp inflorescences produces numerous by-products in the form of waxy material, which are currently lost in processing waste but which contain a mixture of phytocompounds of pharmacological interest, including policosanols.

The Applicant has now surprisingly identified a method for using the aforementioned waxy material normally discarded and extracting policosanols from it, in a mixture with other phyto-compounds or as a fraction with a high degree of purity.

In fact, hemp wax contains policosanols with C20-C36 chains, mainly in fatty acid esterified form. Of particular interest, within the scope of the invention, are policosanols having from 24 to 30 carbon atoms and their mixtures.

In this way it is possible to exploit industrial waste that would otherwise be lost, with a considerable economic impact deriving from the recovery of compounds, the policosanols, which find numerous applications to produce pharmaceutical, nutraceutical, food or cosmetic products.

### Brief description of the drawings

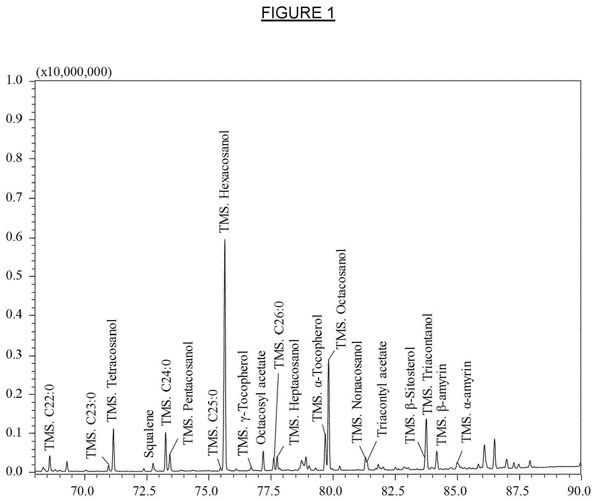

- Figure 1: typical chromatogram, obtained by GC-MS after derivatization, of the wax waste following a saponification reaction carried out in ethanol in the presence of KOH, for the zone in which the policosanols elute.

Therefore, the subject of the present invention is a method for extracting policosanols from industrial hemp processing waste, comprising the following steps:
a) saponification of the waxy material in the presence of an inorganic base;
b) separation of the saponifiable fraction from the unsaponifiable fraction thus formed;
c) isolation of the unsaponifiable fraction containing the policosanols.

The term "processing waste" according to the present invention refers to the waxy material obtained as a by-product of the cannabidiol extraction process from hemp inflorescences.

The waxy material to be subjected to the process of the present invention can be obtained as a by-product of any of the industrial hemp extraction processes known in the art, such as for example in Attard, T. M. et al., Ind. Crops Prod. 2018, 112, 38-46. According to a preferred embodiment of the present invention, the waxy material is obtained starting from inflorescences of industrial hemp, comprising extraction of industrial hemp with an organic solvent and subsequent winterization of the extract thus obtained.

As it is well known to the skilled person, industrial hemp can be subjected to various pre-extraction treatments, such as freeze-drying, decarboxylation, drying, grinding, pellet reduction.

Preferably, the industrial hemp used in the method of the present invention is subjected to freeze-drying.

Any variety of hemp can be used in the method of the invention.

According to a preferred embodiment, the suitable hemp variety is chosen from a monoecious variety or a dioecious variety.

Among the monoecious varieties, Futura 75, Felina 32, Fedora 17 can be mentioned. Examples of dioecious varieties that can be used in the present invention are Kompolti and Finola.

According to the present invention, the freeze-dried hemp is extracted with an organic solvent, preferably an apolar organic solvent, wherein said apolar organic solvent is selected from hexane, C5-C9 hydrocarbon solvents, mixture of hydrocarbons such as petroleum ether, chlorinated solvents such as dichloromethane or chloroform , ketone solvents such as acetone and methylketone, alcohols such as methanol, ethanol or isopropanol, ethers such as diethyl ether or tetrahydrofuran or esters such as ethyl acetate.

An organic solvent particularly suitable for the method of the invention is hexane. The solvent is added in variable quantities of 100-200 ml for every 10 g of initial lyophilizate.

The extraction is carried out at room temperature or under low heating at a temperature between about 18°C and 30°C, depending on the solvent used.

As it is well known to the skilled person, the techniques that can be used to carry out the hemp extraction, are for example soxhlet, mechanical stirring, ultrasound. Preferably, the extraction is carried out by ultrasounds in order to speed up the process.

The extract is subsequently concentrated, preferably at reduced pressure or by a distillation system, and an organic solvent of medium polarity chosen from ethanol, methanol or other short-chain alcohols, preferably ethanol, is added.

The mixture thus obtained is then subjected to the winterization procedure to remove most of the phytocannabinoids present in the matrix, and the waxy material used in the method of the present invention is obtained in the form of a precipitate.

Typically, the waxy material thus obtained contains the following compounds: hydrocarbons, fatty acids, alcohols, aldehydes, phytosterols, fatty acid esters, phytocannabinoids, terpenes, flavonoids, free policosanols and may contain traces of psychoactive compounds, such as for example tetrahydrocannabinol.

According to the method of the invention, the waxy material is first subjected to a saponification reaction (step a).

Said saponification reaction is carried out in an aqueous, alcoholic or hydroalcoholic solution in which the percentage of water present in the reaction mixture is in the range of 40-90% by weight, preferably 50%.

In the case of hydrolysis carried out in alcoholic and hydroalcoholic solutions that do not satisfy this percentage, it is preferable according to the present invention to proceed with the addition of a saline solution, more preferably a saturated solution of NaCl.

In the case of an alcoholic or hydroalcoholic solution, the alcohol used is preferably 96% ethanol.

The saponification reaction is then carried out in the presence of an inorganic base, preferably hydroxides of alkali or alkaline-earth metals, more preferably potassium hydroxide at a temperature between 65°C and 100°C.

According to a preferred embodiment, the weight ratio of waxy material / inorganic base is comprised between 1.5 and 3, more preferably about 1.8.

The saponification reaction therefore leads to the formation of two fractions, namely a saponifiable fraction and an unsaponifiable fraction. The saponifiable fraction consists of fatty acids and esters, while the unsaponifiable fraction consists of hydrocarbons, alcohols, aldehydes, phytosterols, phytocannabinoids, terpenes, policosanols.

In step b) of the method of the invention, the unsaponifiable fraction, which contains the policosanols of interest, is separated from the saponifiable fraction by techniques well known to those skilled in the art, such as for example filtration or liquid-liquid extraction with an organic solvent immiscible with water, preferably ethyl ether. Other solvents immiscible with water can be used such as C5-C9 hydrocarbon solvents, a mixture of hydrocarbons such as petroleum ether, chlorinated solvents such as dichloromethane or chloroform, esters such as ethyl acetate.

After isolation of the unsaponifiable fraction (step c), a mixture of phytocompounds containing about 3-5% of policosanols is obtained, which can optionally be enriched in policosanols, by means of an appropriate purification technique, such as for example recrystallization from a solvent, or a chromatographic technique, preferably chromatography on silica, in order to obtain a mixture of C24-C30 policosanols having a purity of about 95-99%, in which hexacosanol (C26) is the predominant compound. In particular, hexacosanol is present in the mixture of phytocompounds in a percentage between 23-43%.

This further purification allows to eliminate most of the co-extracted compounds and residual phytocannabinoids.

The extraction process of the present invention is also scalable at an industrial level and the solvents used can be advantageously recovered by distillation.

The qualitative and quantitative characterization of the composition of the extracts in step c) or after possible purification can be evaluated with techniques known in the literature, in particular by gas chromatography combined with flame induction detector or mass spectrometry, after derivatization as acetates or silyl derivatives. Therefore, with the method of the present invention it is possible to obtain a mixture of phytocompounds containing policosanols having from 24 to 30 carbon atoms, or a fraction of policosanols with a degree of purity between 95% and 99%, if the mixture is further subjected to a purification step by means of one of the purification processes known in the art such as, for example, recrystallization from solvent or chromatographic separation, preferably chromatography on silica.

The mixture of policosanols obtained through the process of the invention has the following percentage abundances by weight: 9-23% tetracosanol (C24), 23-43% hexacosanol (C26), 2-22% heptacosanol (C27), 15-40% octacosanol (C28), 6-29% triacontanol (C30).

The mixture of policosanols, obtained by the method of the present invention, can be used as such as an ingredient in pharmaceutical, nutraceutical, food or cosmetic products.

Alternatively, it can be subjected to a further purification process known in the art for the isolation of a single policosanol or mixture of policosanols enriched in a particular policosanol to be used as an ingredient in pharmaceutical, nutraceutical, food or cosmetic products.

### Experimental part

### Example 1

The wax, a by-product of the industrial extraction of cannabidiol, was subjected to trans-esterification according to the following procedure: 10 g of wax was dispersed in 50 ml of ethanol in the presence of 5.6 g of KOH. The reaction was carried out under magnetic stirring at 78°C for 6 h. The resulting mixture was extracted with 30 ml of diethyl ether. The organic phase was washed with 10 ml of water, then dried over anhydrous Na₂SO₄ (typically 10 g). The Na₂SO₄ was filtered and removed, then the extract was evaporated in a rotary evaporator. The residue was solubilized in 5 ml of chloroform and the composition was determined by GC-MS and GC-FID analysis (quantitative analysis) after silylation (Figure 1 shows a typical chromatogram of the unsaponifiable fraction obtained from the wax waste). The composition of this extract typically contains cannabidiol, policosanols and lipophilic compounds (mainly tocopherols, fatty acids, other cannabinoids, ethyl esters of fatty acids, terpenes and terpenoids).

The mixture of this example contained 4.3% policosanols, according to the following % abundance reported below in Table 1:

**Table 1: percentage composition of policosanols in the unsaponifiable fraction obtained from the wax waste.**

| **Compound** | **Abundance %** |
|---|---|
| tetracosanol (C24) | 0.4 |
| pentacosanol (C25) | 0.24 |
| hexacosanol (C26) | **2.78** |
| heptacosanol (C27) | 0.14 |
| octacosanol (C28) | 0.35 |
| nonacosanol (C29) | 0.12 |
| triacontanol (C30) | 0.25 |

The remaining components are phytocannabinoids (85%, of which 82.8% cannabidiol), fatty acids (2.5%), tocopherols (1%), and 7.2% of other compounds (phytosterols, policosanol esters, terpenes, ethyl esters of fatty acids).

### Example 2

In a typical laboratory-scale procedure, inflorescences of industrial hemp (50 g) are freeze-dried and placed in an ultrasonic bath for 30 min with hexane (600 ml). In this example, a sample of Finola hemp (Finnish dioecious variety) was used. The mixture was centrifuged at 8000 × g for 10 min. The extraction was repeated and the supernatants combined. At the end of the extraction process, the hexane was removed by means of a rotary evaporator at room temperature. The oily residue obtained was dissolved in 40 ml of hot ethanol (about 75°C) and subsequently placed at -20°C for 48 h in order to induce precipitation of the wax by means of a winterization procedure. The wax was isolated by centrifugation at 4°C, 8000 × g for 30 min. The solvent was removed. The residual wax was washed with 5 ml of ethanol. The washing was discarded. The recovery of this process was calculated as the ratio of the weight of the wax to the weight of the starting material (20%).

The wax obtained was subjected to saponification. 10 g of wax was dispersed in 50 ml of ethanol in the presence of 5.6 g of KOH. The reaction was carried out under magnetic stirring at 70°C for 6h. 50 ml of saturated solution of NaCl in water were added to the reaction crude and the resulting mixture was extracted with 50 ml diethyl ether three times. The combined organic phases were dried on 10 g anhydrous Na₂SO₄, then evaporated in a rotary evaporator. The mixture obtained contains about 280 mg of policosanols with a yield by weight of 2.8% with respect to the starting wax. The quantitative analysis of the main policosanols is shown in Table 2.

**Table 2: Yield, in milligrams, of the main policosanols extracted from 10 g of wax from industrial hemp Finola variety.**

| **Compound** | **mg** |
|---|---|
| tetracosanol (C24) | 36 |
| hexacosanol (C26) | 83 |
| heptacosanol (C27) | 26 |
| octacosanol (C28) | 68 |
| triacontanol (C30) | 66 |
| total | 279 |

### Example 3

10 g of hemp inflorescence (mixture in equal parts of three French monoecious varieties, namely Futura 75, Felina 32, Fedora 17, and two dioecious varieties, namely the Hungarian Kompolti and the Finnish Finola) were extracted as described in the example 2, then the mixture of the unsaponifiable fraction was further purified for the isolation of a pure fraction of policosanols. In this example, the mixture was purified on silica by normal phase chromatography with isocratic elution. The unsaponifiable fraction was purified on 40 g of silica and fractionated by eluting with dichloromethane.

Collecting 10 fractions of 80 ml, policosanols are found in fractions 4-6, which are devoid of the presence of phytocannabinoids. The obtained mixture contains about 4.8 mg of policosanols with a purity of 99%. The percentage composition is shown in Table 3.

**Table 3: Yield, in micrograms, of the main policosanols extracted from 10 g of industrial hemp and purified by normal phase chromatography on silica and isocratic elution with dichloromethane.**

| **Compound** | **Composition %** |
|---|---|
| tetracosanol (C24) | 21 |
| hexacosanol (C26) | 27 |
| heptacosanol (C27) | 21 |
| octacosanol (C28) | 20 |
| triacontanol (C30) | 11 |

## Claims

1. Method for the extraction of policosanols from industrial hemp processing waste, comprising the following steps:
a) saponification of a waxy material in the presence of an inorganic base;
b) separation of the saponifiable fraction from the unsaponifiable fraction thus obtained;
c) isolation of the unsaponifiable fraction containing policosanols.

2. Method according to claim 1, **characterized in that** it comprises one or more further purification steps.

3. Method according to any one of the preceding claims, **characterized in that** said waxy material is obtained starting from industrial hemp inflorescences, by extraction of industrial hemp with an organic solvent and subsequent winterization of the extract obtained.

4. Method according to claim 3, **characterized in that** the industrial hemp is selected among a monoecious variety or a dioecious variety.

5. Method according to any one of claims 3-4, **characterized by** the fact that industrial hemp is selected from Futura 75, Felina 32, Fedora 17, Kompolti and Finola.

6. Method according to any one of the preceding claims, **characterized in that** the weight ratio between waxy material / inorganic base is comprised between 1.5 and 3, preferably of about 1.8.

7. Method according to any one of the preceding claims, **characterized in that** said separation of the saponifiable fraction from the unsaponifiable fraction occurs in the presence of a solvent immiscible with water, preferably ethyl ether.

## Patentansprüche

1. Verfahren zur Extraktion von Polikosanolen aus Industriehanfverarbeitungsabfällen, umfassend die folgenden Schritte:
a) Verseifung eines wachsartigen Materials in Gegenwart einer anorganischen Base;
b) Abtrennen der verseifbaren Fraktion von der derart erhaltenen unverseifbaren Fraktion;
c) Isolierung der unverseifbaren Fraktion, die Polikosanole enthält.

2. Verfahren nach Anspruch 1, **dadurch gekennzeichnet, dass** es einen oder mehrere weitere Reinigungsschritte umfasst.

3. Verfahren nach einem der vorhergehenden Ansprüche, **dadurch gekennzeichnet, dass** das wachsartige Material ausgehend von Industriehanfblütenständen durch Extraktion von Industriehanf mit einem organischen Lösungsmittel und anschließende Winterisierung des erhaltenen Extrakts erhalten wird.

4. Verfahren nach Anspruch 3, **dadurch gekennzeichnet, dass** der Industriehanf aus einer mono- oder diözischen Sorte ausgewählt wird.

5. Verfahren nach einem der Ansprüche 3-4, **dadurch gekennzeichnet, dass** der Industriehanf aus Futura 75, Felina 32, Fedora 17, Kompolti und Finola ausgewählt wird.

6. Verfahren nach einem der vorhergehenden Ansprüche, **dadurch gekennzeichnet, dass** das Gewichtsverhältnis zwischen wachsartigem Material/anorganischer Base zwischen 1,5 und 3, vorzugsweise etwa 1,8, umfasst.

7. Verfahren nach einem der vorhergehenden Ansprüche, **dadurch gekennzeichnet, dass** die Trennung der verseifbaren Fraktion von der unverseifbaren Fraktion in Gegenwart eines mit Wasser nicht mischbaren Lösungsmittels, vorzugsweise Ethylether, erfolgt.

## Revendications

1. Procédé pour l'extraction de policosanols à partir de déchets de traitement de chanvre industriel, comprenant les étapes suivantes :
a) la saponification d'une matière cireuse en présence d'une base inorganique ;
b) la séparation de la fraction saponifiable de la fraction insaponifiable ainsi obtenue ;
c) l'isolement de la fraction insaponifiable contenant des policosanols.

2. Procédé selon la revendication 1, **caractérisé en ce qu'**il comprend une ou plusieurs étapes supplémentaires de purification.

3. Procédé selon l'une quelconque des revendications précédentes, **caractérisé en ce que** ladite matière cireuse est obtenue à partir d'inflorescences de chanvre industriel, par extraction de chanvre industriel avec un solvant organique et hivernage ultérieur de l'extrait obtenu.

4. Procédé selon la revendication 3, **caractérisé en ce que** le chanvre industriel est choisi parmi une variété monoïque ou une variété dioïque.

5. Procédé selon l'une quelconque des revendications 3 à 4, **caractérisé par le fait que** le chanvre industriel est choisi parmi Futura 75, Felina 32, Fedora 17, Kompolti et Finola.

6. Procédé selon l'une quelconque des revendications précédentes, **caractérisé en ce que** le rapport pondéral matière cireuse/base inorganique est compris entre 1,5 et 3, de préférence d'environ 1,8.

7. Procédé selon l'une quelconque des revendications précédentes, **caractérisé en ce que** ladite séparation de la fraction saponifiable de la fraction insaponifiable se fait en présence d'un solvant non miscible à l'eau, de préférence l'éther éthylique.
